(19) Europäisches Patentamt
European Patent Office
Office européen des brevets

(11) EP 2 051 691 B1

(12) **EUROPEAN PATENT SPECIFICATION**

(45) Date of publication and mention of the grant of the patent:
**19.05.2010 Bulletin 2010/20**

(21) Application number: **06792426.6**

(22) Date of filing: **13.10.2006**

(51) Int Cl.:
***A61K 8/36*** (2006.01) ***A61K 8/63*** (2006.01)
***A61K 8/68*** (2006.01) ***A61K 8/86*** (2006.01)
***A61Q 19/00*** (2006.01) ***A61Q 19/08*** (2006.01)

(86) International application number:
**PCT/EP2006/009919**

(87) International publication number:
**WO 2008/043386 (17.04.2008 Gazette 2008/16)**

(54) **SKIN TREATMENT COMPOSITION**

HAUTBEHANDLUNGSZUSAMMENSETZUNG

COMPOSITION DE TRAITEMENT CUTANÉ

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**29.04.2009 Bulletin 2009/18**

(73) Proprietor: **Evonik Goldschmidt GmbH**
**45127 Essen (DE)**

(72) Inventors:
- **KOREVAAR, Cornelis Gerrit Nijs**
  **2341 KB Oegstgeest (NL)**
- **FARWICK, Mike**
  **45138 Essen (DE)**
- **MEYER, Jürgen**
  **45134 Essen (DE)**
- **LERSCH, Peter**
  **46537 Dinslaken (DE)**
- **REIDICK, Wilma**
  **46236 Bottrop (DE)**
- **WEITEMEYER, Christian**
  **45257 Essen (DE)**

(56) References cited:
**WO-A-94/00127** **WO-A-99/29293**
**WO-A-03/013460** **WO-A-2006/053912**
**US-A1- 2005 089 500**

- **DEGUSSA: "Skinmimics"[Online] May 2003 (2003-05), XP002439025 Retrieved from the Internet: URL:www.degussa-personal-care.com>**
- **M. DE JAGER ET AL.: "Medelling the stratum corneum lipid organisation with synthetic lipid mixtures: the importance of synthetic ceramide composition" BIOCHIMICA ET BIOPHYSICA ACTA, vol. 1684, 2004, pages 132-140, XP002439026**
- **J. BOUWSTRA ET AL.: "The role of ceramide composition in the lipid organisation of the skin barrier" BIOCHIMICA ET BIOPHYSICA ACTA, vol. 1419, 1999, pages 127-136, XP002439027**
- **M. DE JAGER: "Novel lipid mixtures based on synthetic ceramides reproduce the unique stratum corneum lipid organisation" JOURNAL OF LIPID RESEARCH, vol. 45, 2004, pages 923-932, XP002439028**
- **DIETZ T ET AL: "FORMULIERUNG VON CERAMIDEN - EINE GRUNDLEGENDE UNTERSUCHUNG" SOFW-JOURNAL SEIFEN, OELE, FETTE, WACHSE, VERLAG FUR CHEMISCHE INDUSTRIE, AUGSBURG, DE, vol. 129, no. 5, 2 May 2003 (2003-05-02), pages 2-4,6, XP001159584 ISSN: 0942-7694 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

Field of the Invention

This invention relates to topical compositions consisting of lipids incorporated into lamellar structures preventing crystallisation for optimised bioavailability and application to human skin.

Background and Prior Art

Skin is a highly complex tissue acting as a protector against physical, chemical and biological attacks. It plays a crucial role in the protection against dehydration and the control of body temperature (A Short Textbook of Cosmetology, KF de Polo (Ed.), Verlag für chemische Industrie, H. Ziokowski GmbH, Augsburg (D), 1998). This barrier is provided by the "horny layer" (stratum corneum, SC), representing the outermost layer of the epidermis. The horny layer is a thin inert, water-retaining barrier which both regulates the moisture content of the skin and protects it against external influences.

Due to its structure it is often compared to a brick wall in which the nonviable corneocytes are embedded like bricks in a matrix of lipids ("Mortar") (Elias PM, J. Invest. Dermatol. 80 (Suppl 1), 44 (1983)). The lipid mixture is assembled into densely packed lamellar structures consisting of ceramides, cholesterol and fatty acids. In the literature different compositions of skin lipids are given depending on extraction and analytical methods exerted and the origin of the skin used for analysis. On a weight basis, these lipids constitute of approximately 47 % by weight ceramides, 24 % by weight cholesterol, 11 % by weight free fatty acids and 18 % by weight cholesterol esters (Rawlings AV, Int. J. Cosmet. Sci. 25, 1-33 (2003)).

The lipid environment of the stratum corneum is an essential factor for maintaining the skin's equilibrium. As a result of age, health or environmental conditions, changes in the lipid composition occur, leading to a weakening of the barrier function (Rawlings, A.V. et al., J.Invest.Dermatol. 103, 731-740 (1994); Motta S;Arch Dermatol. 1994; 130, 452-456; Choi et al, J Invest Dermatol. 2005 Mar.,124(3), 587-595). These findings lead to the concept that ceramides in combination with cholesterol and fatty acids are valuable components of skin care products, since the topical application of such products can replenish low levels of stratum corneum lipids.

It is well known in the literature that the topical application of lipid mixtures containing ceramides, fatty acids and cholesterol improves the skin performance under various suboptimal conditions, e.g. barrier recovery in chronologically aged skin (Zettersten et.al., J Am Acad Dermatol. 1997 Sep.,37, 403-408), in psychologically stressed skin (Choi et al, J Invest Dermatol. 2005 Mar.,124(3), 587-595) or improving the barrier properties and the clinical condition of the skin in contact dermatitis (Beradesca et.al., Contact Dermatitis, 2001, 45, 280-285).

EP 0 644 764 (Elias et.al.) describes specific lipid mixtures consisting of acylceramides, ceramides or glucosylceramides together with cholesterol and/or fatty acids for epidermal moisturization and repair of barrier function. The invention is related to the treatment of skin diseases which display hyperproliferation and disruptions of the barrier function.

The activity of short chain ceramides on keratinocyte proliferation and differentiation has been described in the literature (Pillai et al. J Invest Dermatol. Symp. Proceed.1, 39-93). US 5,578,641 (Jackson et. al.) described the topical applications of one or more ceramide pathway intermediates or precursors for the supplementation of skins own ceramide production pathways in the epidermis. Listed structures include free sphingoid bases and their N-acyl derived ceramides with a carbon chain length of 1 to 10 atoms. The invention is related to the treatment of dry and/or (photo-)damaged skin for reducing or delaying wrinkle formation.

EP 0 975 325 (Lambers) describes compositions comprising a combination of a free sphingoid base and a ceramide showing a positive synergistic effect on lipid barrier function.

The physical organization of the membrane bilayer structure is crucial for an effective skin barrier and this is provided by a lipid lamellar assembly in a tightly packed orthorhombic configuration. Detailed research on the assembly of SC lipids has been performed establishing the so-called "Sandwich model" (Bouwstra J et al., Skin Pharmacol. Appl. Skin Physiol. 14, 52-62 (2001)). By electron microscopy and x-ray diffraction the SC lipids are observed as alternating broad/ narrow/ broad sequences of bilayers representing two broad lipid layers with crystalline structure separated by a narrow central lipid layer with fluid domain. The optimal ratio for the topical application of the stratum corneum lipids ceramides, cholesterol and fatty acids is in the range of 1:1:1 on a molar basis. The structural organisation of the stratum corneum lipids *in vivo* can be mimicked by lipid mixtures prepared with well-defined synthetic ceramides 1, 2, 3 and 9 (de Jager et al, J. Lipid Res. 2005 Dec., 46(12), 2649-2656). Using the more recent lettering system of Ceramides classification (Motta et al. BBA, 1993, 1182, 147-151) these Ceramides are called Cer(amide) EOS, Cer NS, Cer NP and Cer EOP respectively.

Although the dermatological importance of ceramides is known, it still remains a challenging target to incorporate ceramides in a stable way in cosmetic formulations. Stable in that respect means that not only the formulation is physically stable but also that ceramides do not recrystallize out of these formulations.

There have been publications explaining how ceramides can be included in cosmetic formulations avoiding this crystallization phenomenon (e.g. T. Dietz, P. Hameyer, SOFW-Journal (5), 2-9 (2003)). In these studies it was shown on the example of Ceramide 3 (NS) that up to 1 % by weight of this ceramide can be included into cosmetic oil-in-water emulsions without crystallization. As a crucial step in successfully formulating ceramides it was pointed out that the ceramides had to be dissolved in the oil phase in a first processing step. In order to achieve that the oil phases had to be heated to 90°C.

Unfortunately, such a processing is not possible for many types of cosmetic formulations due to limitations in available processing equipment or due to restrictions caused by temperature sensitive ingredients in the formulations. Also for ecological and economical (longer processing, costs for heating) reasons such a way of processing is not optimal.

Moreover, in the Dietz paper it has also been described that all attempts to include Ceramide 3 (Cer NS) in a stable way into water-in-oil emulsions failed. Ceramide 3 (Cer NS) crystals appeared in all formulations after a few days of storage at room temperature even at very low ceramide concentrations of 0.1 % by weight in these formulations.

A limitation of stable inclusion of ceramides in formulations just to oil-in-water emulsions is a significant restriction for cosmetic formulators as water-in-oil emulsions are known for their moisturizing benefits especially for dry skin.

Additionally it is even more challenging to include ceramides with N-acyl side chains that are longer than 18 Carbon atoms stable into formulations as these long-chain ceramides tend to crystallize much easier.

However, in order to provide a mixture of ceramides / cholesterol / fatty acids that is able to mimic skin identical lamellar structures, Bouwstra and her group showed that especially such long-chain ceramides are needed (Bouwstra, J.A. et al. J.Lipid Research 1998, 39, 186-196; Bouwstra, J.A. et al. J. Invest. Dermatol. 2002, 118, 606-617; de Jager, M.W. et al. Chem.Phys. Lipids 2003, 124, 123-134)

For all these reasons there is a need to offer the cosmetic and pharmaceutical formulator a composition that is able

1) to mimic skin identical lamellar structures, containing long-chain ceramides (N-acyl side chain longer than 18 carbon atoms) without crystallization of ceramides in the composition upon storage,
2) to be incorporated easily into formulations without the necessity of heating the oil phase or water phase to temperatures above 40°C. Therefore the composition needs to be liquid at room temperature and should have no compatibility problems with anionic, amphoteric or cationic ingredients,
3) to allow the stable inclusion of long-chain ceramides into oil-in-water emulsions as well as into water-in-oil emulsions. In both types of emulsion systems the composition should be able to prevent recrystallization of ceramides upon storage.

Surprisingly, it was found that all three targets can be fulfilled by a suitable design of a skin treatment composition based on nonionic hydrophilic emulsifiers, consistency enhancers, specific ceramide mixtures containing medium and long-chain ceramides, fatty acids and cholesterol. This cholesterol can either be animal derived, be produced by a microorganism, have a plant origin or be synthetized starting from plant-derived material.

Additionally, it was surprisingly found that inclusion of short chain ceramides (C4 to C8 alkyl chains) did not change the lamellar lipid organization. This inclusion, however, resulted into an increased production of ceramides in the skin.

Therefore the short-chain ceramides surprisingly acted as regulators when the cosmetic composition was applied onto the skin.

The present invention therefore corresponds to liquid and pumpable skin treatment compositions containing (all percentages are given as weight-%):

A) 0.1 to 10 % by weight preferred 0.2 to 8 % by weight, specially preferred 0.5 to 5 % by weight of a mixture consisting of:

A1) 10 to 80 % by weight of a ceramide or of a mixture of at least two ceramides with the proviso that at least one of these ceramides contains a long alkyl, alkenyl or acyl side chains with 20 to 26 carbon atoms and that less than 40 % by weight has a side chain larger than 26 carbon atoms,
A2) 10 to 45 % by weight of cholesterol,
A3) 10 to 45 % by weight of free fatty acids with C12 to C30 alkyl-, alkenyl-, alkadienyl-, alkatrienyl-, alkapolyenyl chains or combinations thereof;

B) 0.5 to 10 % by weight, preferred 1 to 8 % by weight, specially preferred 4 to 6 % by weight, of a nonionic emulsifier or a nonionic emulsifier mixture with a combined HLB value of 12 to 19;
C) $\geq$ 40% by weight of water and optionally;
D) 0.1 to 10 % by weight, preferred 0.1 to 6 % by weight, specially preferred 1 to 5 % by weight, consistency

enhancers like glyceryl stearate or C16-C22 alkanols and optionally;
E) auxiliaries and additives

with the proviso that A) to E) must add up to 100 % by weight by weight.

The main advantages of the inventive mixture are:

1) has liquid and pumpable / pourable condition at room temperature i.e. about 20°C to 30°C and
2) shows no crystallization of ceramides upon storage for up to 1 year at temperatures between -5°C and 40°C and
3) can easily be incorporated into cosmetic formulations without the necessity of heating the oil phase or water phase to temperatures above 40°C, showing no incompatibility effects with anionic, amphoteric or cationic ingredients and
4) makes the stable inclusion of the long-chain ceramides into oil-in-water as well as into water-in-oil emulsions possible by preventing recrystallization of ceramides in these formulations upon storage for up to 1 year at temperatures between 5°C and 40°C.
5) enables the stable inclusion of specific Ceramides mixtures that are able to mimic skin identical lamellar structures, into oil-in-water as well as into water-in-oil emulsions by preventing recrystallization of ceramides in these formulations upon storage for up to 1 year at temperatures between 5°C and 40°C
6) allows for the addition of short chain Ceramides to these specific Ceramides mixtures without losing any of the advantages mentioned above.

In a preferred embodiment of the invention the skin treatment composition contains 0.5 to 5 % by weight of the ceramide / cholesterol / free fatty acid mixture A) and 4 to 6 % by weight of a nonionic emulsifier or a nonionic emulsifier mixture with a combined HLB value of 12 to 19 and 1 to 5 % by weight consistency enhancers like glyceryl stearate or C16 to C22 alkanols and optionally auxiliaries and additives added up to 100 % by weight by weight with water.

The Ceramide(s) present in mixture A1) consist of:

1) a sphingoid base with a general structure according to formula 1

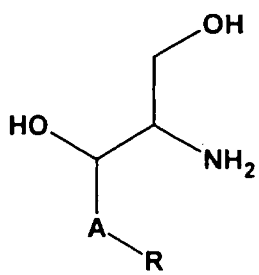

wherein

A is $-CH_2-CH_2-$, $-CH=CH-$, or $-C(H)OH-CH_2-$ and
R is a straight chain or branched alkyl group having 10 to 22 carbon atoms which may optionally contain one or more double bonds and/or may optionally be substituted with one or more hydroxyl groups.

Preferably R is either a straight chain alkyl group having 12 to 18 carbon atoms or C(H)OH coupled to a straight chain alkyl group having 11 to 17 carbon atoms.

Most preferred A and R combinations are Phytosphingosine (P), Sphingosine (S), 6-hydroxysphingosine (H) and Sphinganine (Sa).

The above Sphingoid base is coupled via an amide linkage to a fatty acid (both hydroxy and non hydroxy fatty acids) according to formula 2

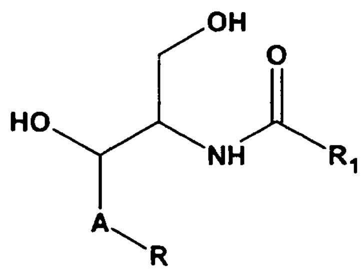

wherein

$R_1$ is a linear or branched alkyl chain containing 1 to 55 carbon atoms, preferably 3 to 50 carbon atoms and more preferably 5 to 44 carbon atoms.

The alkyl chain may be interrupted by an oxygen atom or by an internal ester group; may eventually contain one or more double bonds and may eventually be substituted by one or more hydroxyl groups,
or
skinidentical Ceramide(s). These are Ceramides with a composition as above but with a stereochemical configuration, that is identical to the natural ceramides present in the mammalian skin,
or
natural Ceramide(s). These are Ceramides with a composition as above, which are extracted from the mammalian skin and have maintained their original steriochemical configuration during the extraction process,
or
combinations thereof.

In a preferred embodiment of the invention the ceramide / cholesterol / free fatty acid mixture A contains a combination of natural or skin identical ceramides A1) consisting of:

A1a) a content > 30 % by weight of ceramides with a long alkyl, alkenyl or acyl chain with > C18, while the contents of very long chain (≥C27) acylceramides should be not more than 40% by weight;

A1b) a content of 1 to 30 % by weight by weight of ceramides with a short alkyl chain with C4 to C8.

Mixture A1) present in the composition according to the invention consists preferably of:

- a combination of at least 2 Ceramides with a long alkyl, alkenyl or acyl sidechain, long meaning more than 18 Carbon atoms;

- at least one of these Ceramides should be Ceramide NS, NP, NH with a chain length of 20 to 26 Carbon atoms, and should form at least 40 % by weight of Mixture A1);

- acylceramides like Ceramide EOS, EOP, EOH should not be present in more than 40% by weight of mixture A1);

- part of the Sphingosine, 6-hydroxysphingosine and Phytospingosine derived Ceramides may be exchanged by their Sphinganine derived equivalents;

- optionally Ceramides of medium side chain length (16 to 18 Carbon atoms) can be added to mixture A1) to a maximum of 30 % by weight;

- optionally 1) Ceramides of short side chain length (4 to 8 C atoms) or 2) Sphingoid bases and/or Ceramides with a side chain of 2 Carbon atoms can be added to mixture A1). This to a maximum of 30 % by weight (1) and 15 % by weight.(2) respectively.

In a more preferred embodiment of the invention the ceramides / cholesterol / free fatty acid mixture A contains specific combinations of natural or skin identical ceramides A1, that are able to mimic skin identical lamellar structures (in the following referred to as A1 lam). For this aim Ceramides mixture $A1_{1am}$. should consist of at least 3, but preferably

4 natural or skin-identical ceramides:

- 8 to 35 % by weight preferably 9 to 15 % by weight of one or two Acyl Ceramides with a side chain of ≥ 27 carbon atoms, wherein up to 45 % by weight of the total acyl ceramides can be ceramide 9
- ≥ 10 % by weight, preferably 25 to 40 % by weight of at least one sphingosine based Ceramide NS or AS,
- ≥ 10 % by weight, preferably 25 to 40 % by weight of at least one one phytosphingosine based Ceramide NP or AP, preferably Ceramide 3 (Cer NS) optionally up to 30 % by weight of natural or skin-identical ceramides with a short alkyl side chain (C4 to C8) can be added.

Most preferred compositions of Mixture $A1_{1am}$. being able to mimic skin identical lamellar structures consist of:

- 50 to 70 % by weight of Ceramide NS + Ceramide NP in a 45/55 ratio. Both with a side chain length of 20 to 26 C atoms. Upto 20 % by weight of the NS + NP may be replaced by Ceramide NH with a similar sidechain length;
- 14 to 20 % by weight of Ceramide EOS + Ceramide EOP in a 6 to 4 ratio. Both with side chain lengths between 27 and 32 C atoms. Upto 20 % by weight of the EOS + EOP may be replaced by Ceramide EOH with a similar side chain length;
- ≥ 10 % by weight, preferably 15 % by weight, of Ceramide NP with a medium side chain length of 16 to 18 C atoms. About 30 % by weight of the Ceramide NP may be replaced by its Sphingosine [NS], 6-OH Sphingosine [NH] or Sphinganine [NSa] based equivalent;
- ≥ 0 % by weight, preferably 10 % by weight, of Ceramide AP with a side chain length of 16 to 24 C atoms. About 30 % by weight of the Ceramide NP may be replaced by its Sphingosine [NS], 6-OH Sphingosine [NH} or Sphinganine [NSa] based equivalent;
- optinionally about 10 % by weight of short chain (Side chain of 4 to 8 C atoms) Ceramide NS + NP in a 1/1 ratio may be added to the A1 mixture. Upto 20 % by weight of the NS + NP may be replaced by Ceramide NH or NSa with a similar sidechain length. When using this option the dosages of the medium and long chain Ceramides in mixture $A1_{lam}$. are reduced proportionally.

The free fatty acids A3) present in the composition according to the invention consists preferably of fatty acids with an alkyl, alkenyl-, alkadienyl-, alkatrienyl-, alkapolyenyl- chain of C12 to C30, most preferably of C18 to C26, or combinations thereof. In a preferred embodiment of the invention behenic acid (C22) is used as free fatty acid component A3).

The nonionic emulsifier or emulsifier mixture B) present in the composition according to the invention include compounds from at least one of the following groups:

- addition products of from 2 to 30 mol of ethylene oxide and/or 0 to 5 mol of propylene oxide onto linear fatty alcohols having 8 to 22 carbon atoms, onto fatty acids having 12 to 22 carbon atoms and onto alkylphenols having 8 to 15 carbon atoms in the alkyl group;
- $C_{12/18}$-fatty acid mono- and diesters of addition products of from 1 to 30 mol of ethylene oxide onto glycerol;
- glycerol mono- and diesters and sorbitan mono- and diesters of saturated and unsaturated fatty acids having 6 to 22 carbon atoms and ethylene oxide addition products thereof;
- alkyl mono- and oligoglycosides having 8 to 22 carbon atoms in the alkyl radical and ethylene oxide addition products thereof;
- addition products of from 2 to 200 mol of ethylene oxide onto castor oil and/or hydrogenated castor oil;
- partial esters based on linear, branched, unsaturated or saturated $C_{6-22}$-fatty acids, ricinoleic acid, and 12-hydroxystearic acid and glycerol, polyglycerol, pentaerythritol, dipentaerythritol, sugar alcohols (e.g. sorbitol), alkyl glucosides (e.g. methyl glucoside, butyl glucoside, lauryl glucoside), and polyglucosides (e.g. cellulose).Here, the use of partial esters of glycerol, of polyglycerol and of sorbitol is preferred. These are, for example, glyceryl laurate, polyglyceryl-4 laurate and sorbitan laurate;
- mono-, di- and trialkyl phosphates;
- mixed esters of pentaerythritol, fatty acids, citric acid and fatty alcohol according to DE-11 65 574 and/or mixed esters of fatty acids having 6 to 22 carbon atoms, methylglucose and polyols, preferably glycerol or polyglycerol.

The nonionic emulsifier or emulsifier mixture B) present in the composition according to the invention consists preferably of a nonionic ethoxylated emulsifier or a mixture of nonionic ethoxylated emulsifiers with a total HLB value of 12 to 19, most preferably with a HLB value of 14 to 18.

The HLB value for an ethoxylated emulsifier $E_i$ is typically calculated by the formula (1)

$$(1) \quad HLB_i = \frac{E_i}{5}$$

with $E_i$ being the weight percentage of ethylene oxide groups in the ethoxylated emulsifier i. When an emulsifier mixture is used, the total HLB value ($HLB_{mixture}$) is calculated as the simple linear mass weighted average as given by formula (2)

$$(2) \quad HLB_{mixture} = \sum_i [(\frac{m_i}{m_{total}}) \cdot HLB_i]$$

with ($m_i/m_{total}$) being the mass fraction of the emulsifier i.

This type of calculation of the HLB value of nonionic emulsifiers and emulsifier mixtures is based on empirical equations derived by Griffins (W.C. Griffin, J. Soc. Cosmet. Chem., 1949, 1, 311 and W.C. Griffin, J. Soc. Cosmet. Chem., 1954, 5, 249). Alternative calculations of the HLB value are summarized in several text books on emulsion science (e.g. B. P. Binks (edt.), Modern Aspects of Emulsion Science, The Royal Society of Chemistry, Cambridge, 1998).

In a preferred embodiment of the invention Ceteareth-25 (HLB = 16.2) is used as hydrophilic nonionic emulsifier component B).

As auxiliaries and additives G according to the invention all auxiliaries and additives customary in cosmetic and pharmaceutical applications and known to the person skilled in the art can be used. These include, for example, additional consistency regulators, thickeners, waxes, UV photoprotective filters, antioxidants, hydrotropes, preservatives, perfume oils, dyes and additional biogenic active ingredients as described for example in DE 10 2005 011 785.6.

As explained above, the liquid and pumpable skin treatment composition can easily be incorporated in cosmetic, dermatological or pharmaceutical formulations in a cold process.

The cosmetic or dermatological formulation can be an aqueous solution, a water-in-oil (W/O) emulsion, an oil-in-water (O/W) emulsion, an aqueous or a water-alcohol gel, a wet-wipe or an aerosol. In case a hot process is needed to prepare the desired formulation type, e.g. solid sticks or wax containing emulsions, the liquid and pumpable skin treatment composition can also be used for such type of hot processing.

The cosmetic or dermatogical formulation is preferably a W/O or an O/W emulsion that contains 1 to 50 % by weight of an oil phase and 47 to < 99 % by weight water, with respect to the weight of the whole formulation. The oil phase can contain all types of cosmetic emollients known to the person skilled in the art. The emulsions are stabilized by all types of emulsifiers, stabilizing polymers and thickeners known to the person skilled in the art. Examples for such emollients, emulsifiers, stabilizing polymers and thickeners are described in DE 10 2005 011 785.6.

Therefore, the invention further provides cosmetic, dermatological or pharmaceutical preparations which comprise a ceramide containing skin treatment composition according to the invention.

In an embodiment of the invention the skin treatment composition is included in a topical skin treatment formulation with an effective amount between 0.001 and 20 % by weight. In a preferred embodiment of the invention the skin treatment composition is included in a topical skin treatment formulation with an effective amount between 0.05 and 10 % by weight in order to maximize benefits at minimum costs.

Besides the skin treatment composition other specific skin-benefit actives such as anti-ageing actives, moisturizers, sunscreens, skin lightening agents, skin tanning agents may also be included.

Typical additional bioactive compounds are:

- vitamins and derivatives thereof like tocopherol, ascorbic acid, niacinamide, retinol, panthenol;
- antioxidants like alpha-lipoic acid, Coenzyme Q10, idebenone, polyphenolics, flavonoids, stilbens, hydroxystilbens, xanthones or isoflavones;
- anti-inflammatories like bisabolol, allantoin, phytantriol, phytosphingosine, sphingosine;
- moisturising agents, amino acids, hyaluronic acid, polyglutamic acid, trimethylglycine, myoinositol, pyroglutamatic acid, taurine, guanidine and hydroxy acids;
- anti-ageing actives like peptides, modified peptides, proteinhydrolysates, lysophospholipids, beta-glucans, creatine, alpha hydroxy acids, beta hydroxy acids, plant extracts or microbial extracts;
- anti-cellulite agents like caffeine or carnitine;
- skin whitening agents like kojic acid, arbutin, vitamin C and derivatives, hydroquinone, creatinine;
- skin tanning agents like dihydroxyacetone, erythrulose or N-acetyl tyrosin.

In addition, auxiliaries and additives customary in cosmetic and pharmaceutical applications and known to the person skilled in the art can be used. These include, for example,co-emulsifiers, consistency regulators, thickeners, waxes, organic and inorganic UV filters, pigments, buffers, hydrotropes, deodorant and antiperspirant active ingredients, insect repellents, antioxidants, self-tanning agents, preservatives, perfume oils and dyes (as described for example in DE 10 2005 011 785.6).

In addition to topical skin treatment formulations the skin treatment composition can also be incorporated into hair care formulations such as, for example, shampoos and conditioners, where it can show a stimulating effect on the scalp performance.

Another embodiment of the invention is therefore the use of the liquid and pumpaple skin treatment composition for hair care applications.

The liquid and pumpable skin treatment compositions according to the invention can also be used in so-called "wash-off" products, e.g., body wash formulations or bath or shower gels.

Therefore, another embodiment of the invention is the use of the liquid an pumpable skin care treatment in "wash-off" applications.

To prepare formulations containing the liquid and pumpable skin treatment composition according to the present invention, the usual manner for preparing such formulations may be employed. The formulations containing the inventive skin treatment composition may be packaged in any suitable manner such as in a jar, a bottle, tube, roll-ball, or the like, in the conventional manner.

Skin is subject to deterioration through environmental abuse (wind, air conditioning, central heating, pollution, sun exposure etc.) accompanied by the passage of time (chronological ageing, dermatological disorders, hormonal changes). These factors lead to decreased skin performance that manifest themselves among others by dry, rough and rigid skin. These effects are observable *in vivo* by parameters like skin moisture (corneometer measurement), barrier function (transepidermal waterloss measurement) or elasticity (cutometer measurement). Concomitantly to the physiologically observable parameters, there are also processes involved on a molecular level:

- expression of genes involved in differentiation and built up of structural and enzymatic components of the skin barrier (e.g. Fillagrin, Loricrin, Involucrin, Transglutaminase);
- expression of genes involved in the lipid components of the barrier, especially sphingolipid biosynthesis (e.g. Serine-Palmitoyl Transferase and Glucosyl-Ceramide Transferase);
- expression of genes responsible for the formation of tight junctions as a separation layer between the viable epidermis and the cornified Stratum Corneum (e.g. claudin-1) and the water transport in the viable layers of the epidermis (e.g. aquaporin-3).

Cosmetic products which treat or delay the visible signs of ageing and improve the environmental protection should increase the expression marker of genes mentioned above leading to a measurable improvement of classical skin performance parameters.

Dermatological studies revealed that the application of cosmetic formulations containing the skin treatment compositions showed such significant increase of these expression markers.

Examples

The following example emulsions serve to illustrate the subject-matter of the invention in more detail without limiting it to these examples. The concentration data in all examples are given as % by weight.

Examples for liquid and pumpaple skin treatment compositions

The examples STC 1 to 10 illustrate liquid and pumpable skin treatment compositions according to the invention. The skin treatment compositions are prepared by heating the oil phase to 90 to 120°C depending on the Ceramides present (in order to be above the melting point of the dosed ceramides) and heating the water phase to 90°C. Then both phases are combined and homogenized for a short time. Liquid and pumpable skin treatment compositions are obtained that can easily be incorporated into e.g. cosmetic formulations.

| | **STC 1** | **STC 2** | **STC 3** | **STC 4** | **STC 5** |
|---|---|---|---|---|---|
| **Oil Phase** | | | | | |
| Ceteareth-25 | 6.00 % | 5.00 % | 4.00 % | 6.00 % | 4.00 % |
| Glyceryl Stearate | | | 2.00 % | | 2.00 % |

(continued)

| | STC 1 | STC 2 | STC 3 | STC 4 | STC 5 |
|---|---|---|---|---|---|
| **Oil Phase** | | | | | |
| Stearyl Alcohol | 2.00 % | 3.00 % | 2.00 % | 2.00 % | 2.00 % |
| Behenic Acid | 0.51 % | 0.51 % | 0.51 % | 0.77 % | 0.77 % |
| Cholesterol [3)] | 0.50 % | 0.50 % | 0.50 % | 0.75 % | 0.75 % |
| Ceramide 1 (EOS) $C_{27-32}$ [4)] | 0.094 % | 0.094 % | 0.094 % | 0.142 % | 0.142 % |
| Ceramide 9 (EOP) $C_{27-32}$ [4)] | 0.058 % | 0.058 % | 0.058 % | 0.088 % | 0.088 % |
| Ceramide 2 (NS) $C_{22}$ [5)] | 0.276 % | 0.276 % | 0.276 % | 0.414 % | 0.414 % |
| Ceramide 3 (NP) $C_{22}$ [5)] | 0.329 % | 0.329 % | 0.329 % | 0.494 % | 0.494 % |
| Ceramide 3 (NP) $C_{16}$ [5)] | 0.154 % | 0.154 % | 0.154 % | 0.231 % | 0.231 % |
| Ceramide 6 (AP) $C_{18}$ [5)] | 0.089 % | 0.089 % | 0.089 % | 0.134 % | 0.134 % |
| Ceramide 3 (NP) $C_6$ [5)] [= n-Hexanoyl Phytosphingosine] | 0.05 % | 0.05 % | 0.05 % | 0.05 % | 0.05 % |
| Ceramide 2 (NS) $C_6$ [5)] [= n-Hexanoyl Sphingosine] | 0.05 % | 0.05 % | 0.05 % | 0.05 % | 0.05 % |
| Oxynex® LM [1)] | 0.10 % | 0.10 % | 0.10 % | 0.10 % | 0.10 % |
| **Water Phase** | | | | | |
| Glycerin | 3.00 % | 3.00 % | 3.00 % | 3.00 % | 3.00 % |
| Euxyl® K 300[2)] | 1.00 % | 1.00 % | 1.00 % | 1.00 % | 1.00 % |
| Water | ad 100 % | ad 100 % | ad 100 % | ad 100 % | ad 100 % |
| Consistency | thin liquid | thin liquid | liquid | thin liquid | liquid |
| Crystals | No | no | no | no | no |
| Stability | Stable | stable | stable | stable | stable |

1) Oxynex® LM (Merck): or alternative antioxidant mixture
2) Euxyl® K 300 (Schülke & Mayr): preservative mixture
3) Cholesterol either animal or plant based
4) $C_{27-32}$ means with a fatty acid side chain length of 27 to 32 Carbon atoms
5) $C_n$ means with a fatty acid chain length of about n Carbon atoms

| | STC 6 | STC 7 | STC 8 | STC 9 | STC 10 |
|---|---|---|---|---|---|
| **Oil Phase** | | | | | |
| Ceteareth-25 | 6.00 % | 6.00 % | 5.00 % | 4.00 % | 6.00 % |
| Glyceryl Stearate | | | | 2.00 % | |
| Stearyl Alcohol | 2.00 % | 2.00 % | 3.00 % | 2.00 % | 2.00 % |
| Behenic Acid | 0.51 % | 0.51 % | 0.51 % | 0.51 % | 0.77 % |
| Cholesterol | 0.15 % | 0.30 % | 0.30 % | 0.15 % | 0.23 % |
| Ceramide 1 (EOS) $C_{27-32}$ [4)] | 0.094 % | 0.094 % | 0.094 % | 0.094 % | 0.142 % |
| Ceramide 9 (EOP) $C_{27-32}$ [4)] | 0.058 % | 0.058 % | 0.058 % | 0.058 % | 0.088 % |
| Ceramide 2 (NS) $C_{22}$ [5)] | 0.276 % | 0.276 % | 0.276 % | 0.276 % | 0.414 % |
| Ceramide 3 (NP) $C_{21}$ [5)] | 0.329 % | 0.329 % | 0.329 % | 0.329 % | 0.494 % |
| Ceramide 3 (NP) $C_{16}$ [5)] | 0.154 % | 0.154 % | 0.154 % | 0.154 % | 0.231 % |

(continued)

| | STC 6 | STC 7 | STC 8 | STC 9 | STC 10 |
|---|---|---|---|---|---|
| **Oil Phase** | | | | | |
| Ceramide 6 (AP) $C_{18}$ 5) | 0.089 % | 0.089 % | 0.089 % | 0.089 % | 0.134 % |
| Ceramide 3 (NP) $C_6$ 5) [=n-Hexanoyl phytosphingosine] | 0.05 % | 0.05 % | 0.05 % | 0.05 % | 0.05 % |
| Ceramide 2 (NS) $C_6$ 5) [= n-Hexanoyl Sphingosine] | 0.05 % | | 0.05 % | 0.05 % | 0.05 % |
| Oxynex® LM 1) | 0.10 % | 0.10 % | 0.10 % | 0.10 % | 0.10 % |
| **Water Phase** | | | | | |
| Glycerin | 3.00 % | 3.00 % | 3.00 % | 3.00 % | 3.00 % |
| Euxyl® K 300 2) | 1.00 % | 1.00 % | 1.00 % | 1.00 % | 1.00 % |
| Water | ad 100 % | ad 100 % | ad 100 % | ad 100 % | ad 100 % |
| Consistency | thin liquid | thin liquid | thin liquid | thin liquid | liquid |
| Crystals | no | no | no | no | no |
| Stability | stable | stable | stable | stable | stable |

All compositions are stable for at least one year at temperatures from -5 to 40°C. No ceramide crystals can be found in this period for all tested temperatures. This illustrates the excellent storage stability of the skin treatment compositions.

Examples for ceramide / cholesterol / free fatty acid mixtures able to mimic skin identical lamellar structures

Summary Materials and methods SAXD experiments

Methods

All methods used in the SAXD diffraction studies were derived from the work of Prof. Bouwstra and her group. (de Jager et al., J. Lipid Res. 2004, 45, 923 - 932; de Jager et al., J. Lipid Res. 2005, 46,2649 - 2656).

$Al_{lam}$ Mixture Compositions [Sum long+ mid chain Ceramides is 100%]

Natural Ceramides

Pig CER (1-6) mixture, composition conform Bouwstra et al., J. Lipid Res., 1998, 39, 186 to 196 . (Please Note : Composition in %mol instead of % by weight)

Human Cer mixture, conposition conform: Bouwstra et al., J. Invest. Dermatol.,2002, 118, 606-617. (Please Note: Composition in %mol instead of % by weight)

Skin identical Ceramides

SynthCER $II_1$, Composition in mol% (De Jager et al., J. Lipid Res. 2005, 46, 2649-2656)
Ceramide 1 ($C_{30}$) 15 ;Ceramide 2 ($C_{24}$) 51; Ceramide 3 ($C_{24}$)16; Ceramide 3 ($C_{16}$) 9; Ceramide 4 ($C_{24}$) 4;Ceramide 6 ($C_{24}$) 5

SynthCER $II_2$, Composition in mol% (De Jager et al., J. Lipid Res. 2005, 46, 2649-2656)
Ceramide 1 ($C_{30}$) 10 ; Ceramide 9 ($C_{30}$) 5; Ceramide 2 ($C_{24}$) 51; Ceramide 3 ($C_{24}$) 16; Ceramide 3 ($C_{16}$) 9; Ceramide 4 ($C_{24}$) 4;Ceramide 6 ($C_{24}$) 5
Scer6A = STC, This patent

| | Scer6A %mol | Scer6A %w | Scer6A +C2c6C3c6 |
|---|---|---|---|
| Ceramide 1 (EOS) $C_{27-32}$ 4) | 6.25 | 9.4 % | 9.4 % |
| Ceramide 9 (EOP) $C_{27-32}$ 4) | 3.75 | 5.8 % | 5.8 % |

(continued)

| | Scer6A %mol | Scer6A %w | Scer6A +C2c6C3c6 |
|---|---|---|---|
| Ceramide 2 (NS) $C_{22}$ 5) | 28.5 | 27.6 % | 27.6 % |
| Ceramide 3 (NP) $C_{22}$ 5) | 33.0 | 32.9 % | 32.9 % |
| Ceramide 3 (NP) $C_{16}$ 5) | 18.6 | 15.4 % | 15.4 % |
| Ceramide 6 (AP) $C_{18}$ 5) | 9.9 | 8.9 % | 8.9 % |
| Ceramide 3 (NS) $C_6$ 5) [= n-Hexanoyl Phytosphingosine] | 0.0 | 0.0 % | 5.0 % |
| Ceramide 2 (NS) $C_6$ 5) [= n-Hexanoyl Sphingosine] | 0.0 | 0.0 % | 5.0 % |
| 4) $C_{27-32}$ means with a fatty acid side chain length of 27 to 32 Carbon atoms<br>5) $C_n$ means with a fatty acid chain length of about n Carbon atoms | | | |

A2 Cholesterol compositions

Cholesterol

If not mentioned specifically otherwise, animal based cholesterol HP was used, which was supplied by Solvay.Pharmaceuticals, Veenendaal.

Plant derived, semisynthetic Cholesterol

Plant cholesterol provided by Degussa Care Specialties or Syntechol supplied by Sigma.

A3 Free Fatty Acids

FFA mix, composition conform (de Jager et al., J. Lipid Res. 2004, 45, 923 - 932;
Fatty acids C16:0, C18:0, C20:0, C22:0, C23:0, C24:0 and C26:0 were mixed at molar ratios of respectively 1.3, 3.3,6.7, 41.7, 5.4, 36.8 and 4.7 % by weight respectively; FFA, linoleic acid As above but C23:0 replaced by linoleic acid;
FFA, arachidonic acid As above but C23:0 replaced by Arachidonic acid.

Behenic acid [C16 to C24]

Plant derived free fatty acid mixture containing mainly of Behenic acrid (± 85%mol). Further C16:0, C18:0, C20:0, C21:0, C23:0 and C24:0 are present.

Preparation of the mixtures for SAXD diffraction

Samples were prepared with the ceramide mixture described above. The CER mixture was mixed with cholesterol and free fatty acids in equimolar ratio, if not specifically mentioned otherwise. Appropriate amounts of individual lipids dissolved in chloroform:methanol (2:1) were combined to yield mixtures of approximately 1.5 mg total weight at the desired composition with a total lipid concentration of 7 mg/ml. A Camag Linomat IV was used to apply the lipid mixtures on mica. This was done at a rate of 4.3 μl/min under a continuous nitrogen stream. The samples were equilibrated for 10 minutes at the appropriate temperature of 65°C and subsequently hydrated with an acetate buffer of pH 5.0. Finally, the samples were homogenized by 10 successive freeze-thawing cycles between -20°C and room temperature, during which the samples were stored under gaseous argon.

Small angle X-ray diffraction (SAXD)

All measurements were performed at the European Synchrotron Radiation Facility ESRF, Grenoble) using station BM26B. The X-ray wavelength and the sample-to-detector distance were 1.24Å and 1.7m, respectively. Diffraction data were collected with a two-dimensional multiwire gas-filled area detector. The spatial calibration of this detector was

performed using silver behenate. The samples were mounted in a temperature-controlled sample holder with mica windows. The diffraction patterns of the lipid mixtures were obtained at room temperature for a period of 10 minutes.

Small angle X-ray diffraction provides information about the larger structural units in the sample, namely the repeat distance of a lamellar phase. The scattering intensity I (in arbitrary units) was measured as a function of the scattering vector q (in reciprocal nm). The latter is defined as $q = (4n\sin\theta)/\lambda$, in which $\theta$ is the scattering angle and $\lambda$. is the wavelength. From the positions of a series of equidistant peaks ($q_n$), the periodicity, or d-spacing, of a lamellar phase was calculated using the equation $d = 2n\pi / q_n$, $n$ being the order number of the diffraction peak.

Example 1

SAXD patterns with as A1: SynthCerII $_I$ mixture[Fig. 1] and Scer6 mixture [Fig. 2]

Just as published by Prof. Bouwstra and her group SynthCerII$_I$ and Scer6 show SAXD patterns similar to human Cer mixture and pig Cer [1-6] mixture. Compare Fig. 1 and Fig. 2.

Example 2

SAXD patters with as A1 $I_{am}$ mixture:

Scer6
Scer6 + 10 % short chain Ceramides NS + NP
Scer6 FFA $_{\text{linoleic acid}}$

The SAXD patterns show that the formation of the lamellar LPP and SPP structures are not affected by the addition of short chain Ceramides.

Despite the presence of Linoleic acid in the fatty acid mixture the LPP and SPP structures are still formed. The same is true for Arachidonic acid (not shown). Compare Fig. 3

Examples for cosmetical skin care formulations

These examples illustrate the easy use of the skin treatment compositions (STC 1, STC 2 and STC 4) in the preparation of cosmetic skin treatment formulations. The skin treatment compositions can be added to the water phase at room temperature. Depending on the processing the water phase can be processed at room temperature or it can be heated, e.g. to 80°C. Therefore the skin treatment compositions can be used in cold and hot processing of O/W emulsions (formulations 1 to 4 and 5 to 8) and for the processing of W/O emulsions (formulations 9 to 12).

All formulations have been successfully tested on stability for at least six months at temperatures from -5°C to 40°C, additionally for 3 months at 45°C. Freeze stability was successfully tested in 3 freeze-thaw cycles between room temperature and -15°C.

Formulation examples 1 to 4 (cold processed O/W emulsions)

Processing: Phases A and B are combined at room temperature, the emulsion is homogenized. Additional phases are added afterwards.

| | 1 | 2 | 3 | 4 |
|---|---|---|---|---|
| **A (Oil Phase)** | | | | |
| Bis-PEG/PPG-16/16 PEG/PPG-16/16 Dimethicone, Caprylic/Capric Triglyceride | 2.00 % | 2.00 % | | 2.00 % |
| Sorbitan Laurate, Polyglyceryl-4 Laurate, Dilauryl Citrate 4) | | | 1.50 % | |
| Caprylic/Capric Triglyceride | 5.00 % | | | |
| Ethylhexyl Stearate | 3.30 % | 4.00 % | | 5.00 % |
| Ethylhexyl Palmitate | | 4.00 % | 7.00 % | 5.00 % |

(continued)

| | 1 | 2 | 3 | 4 |
|---|---|---|---|---|
| **A (Oil Phase)** | | | | |
| Diethylhexyl Carbonate | 3.30 % | 5.50 % | | 7.50 % |
| Mineral Oil | 5.00 % | | | |
| Octyldodecanol | | 4.00 % | | |
| Decyl Cocoate | | | 3.50 % | |
| Cetearyl Isononaoate | | | 3.00 % | |
| Tocopheryl Acetate | 1.00 % | 0.50% | | 0.50 % |
| **B (Water Phase)** | | | | |
| Skin Treatment Composition 1 (STC 1) | 5.00 % | | 5.00 % | 5.00 % |
| Skin Treatment Composition 2 (STC 2) | | 5.00 % | | |
| Water | 60.80 % | 68.20 % | 75.5 % | 70.80 % |
| Glycerin | 2.00 % | 3.00 % | 3.00 % | 3.00 % |
| Ethanol | 10.0 % | | | |
| Allantoin | 0.10 % | 0.10 % | | |
| Panthenol | 1.00 % | 0.50 % | | |
| Creatine | | 0.30 % | | |
| Carbomer dispersion 1 [5)] | | | 1.40 % | |
| Carbomer dispersion 2 [6)] | | 1.90 % | | |
| Carbomer dispersion 3 [7)] | | | | 1.00 % |
| Xanthan Gum | | 0.10 % | 0.10 % | |
| Polyacrylamide, C13-14 Isoparaffin, Laureth-7 [8)] | 1.50% | | | |
| Polysorbate 80 | | 0.20 % | | 0.20 % |
| **C** | | | | |
| Sodium Hydroxide (10% in water) | | 0.70 % | q.s. | 0 |
| **Z** | | | | |
| Preservative, Parfum | q.s. | q.s. | q.s. | q.s. |
| | | | | |
| Consistency | Lotion | Low viscous lotion | Lotion | Sprayable Lotion |

3) ABIL® Care 85 (Degusssa)
4) TEGO® Care LTP (Degussa)
5) Carbomer dispersion 1 consists of 10 % TEGO® Carbomer 140 (Degussa) and 10 % TEGO® Carbomer 141 (Degussa) dispersed in Ethylhexyl Palmitate
6) Carbomer dispersion 2 consists of 15 % TEGO® Carbomer 141 (Degussa) dispersed in Ethylhexyl Stearate
7) Carbomer dispersion 3 consists of 10 % TEGO® Carbomer 140 (Degussa) and 10 % TEGO® Carbomer 341 ER (Degussa) dispersed in Ethylhexyl Palmitate
8) Sepigel® 305 (Seppic)

Formulation examples 5 to 8 (hot processed O/W emulsions) Processing: Phases A) and B) are heated separately to approx. 80°C. Phase A) is added to phase B) with stirring, a homogenization step follows. The emulsions are cooled to 60°C and phase C) is added, the emulsion is homogenized for a short time. All other phases are added below 40°C.

| | 5 | 6 | 7 | 8 |
|---|---|---|---|---|
| **A (Oil Phase)** | | | | |
| Bis-PEG/PPG-16/16 PEG/PPG-16/16 Dimethicone, Caprylic/Capric Triglyceride | 1.00 % | | | |
| Ceteareth-25 | 1.00 % | | | |
| Polyglyceryl-3 [9)] Methylglucose Distearate | | 3.00 % | | |
| Glyceryl Stearate Citrate 10) | | | 1.50 % | |
| Ceteareth-15, Glyceryl Stearate [11)] | | | | 2.50 % |
| Glyceryl Stearate | 5.00 % | 0.50 % | | |
| Stearyl Alcohol | 2.00 % | 1.00 % | | 1.00 % |
| Ethylhexyl Palmitate | 6.00 % | 4.00 % | 5.00 % | |
| Diethylhexyl Carbonate | 6.00 % | 12.00 % | 6.00% | |
| Caprylic/Capric Triglyceride | 6.00 % | 3.00 % | | |
| Isopropyl Palmitate | | | 2.50 % | |
| Ethylhexyl Stearate | | | | 5.20 % |
| Cetyl Ricinoleate | | | | 3.00 % |
| Isocetyl Palmitate | | | | 2.00 % |
| Dimethicone | | | | 0.50 % |
| Tocopheryl Acetate | | 0.50 % | | |
| **B (Water Phase)** | | | | |
| Skin Treatment Composition 1 (STC 1) | 5.00 % | | | 5.00 % |
| Skin Treatment Composition | | 5.00 % | 5.00 % | |
| 4 (STC 4) | | | | |
| Water | 66.30 % | 66.60 % | 75.10 % | 76.40 % |
| Glycerin | 3.00 % | 3.00 % | 3.00 % | 3.00 % |
| Panthenol | | | 0.50 % | |
| **C** | | | | |
| Carbomer dispersion 4 [12)] | 0.50 % | 1.00 % | | |
| Carbomer dispersion 5 [13)] | | | 1.00 % | 1.00 % |
| **D** | | | | |
| Sodium Hydroxide (10% in water) | 0.20 % | 0.40 % | 0.40 % | 0.40 % |
| **Z** | | | | |
| Preservative, Parfum | q.s. | q.s. | q.s. | q.s. |
| | | | | |

(continued)

| Z | | | | |
|---|---|---|---|---|
| Consistency | Cream | Cream | Lotion | Low viscous lotion |

9) TEGO® Care 450 (Degusssa)
10) AXOL® C 62 (Degussa)
11) TEGO® Care 215 (Degusssa)
12) Carbomer dispersion 4 consists of 20 % TEGO® Carbomer 134 (Degussa) dispersed in Isopropyl Palmitate
13) Carbomer dispersion 5 consists of 20 % TEGO® Carbomer 141 (Degussa) dispersed in Ethylhexyl Palmitate

Formulation examples 9 to 12 (W/O emulsions)

Processing: Heat phase A) to approx. 80°C. Phase B) is added while stirring, a homogenization step follows. The emulsions are cooled to 30°C and phase C) is homogenized again for a short time.

| | 9 | 10 | 11 | 12 |
|---|---|---|---|---|
| **A (Oil Phase)** | | | | |
| Polyglyceryl-4 Diisostearate / Polyhydroxystearate / Sebacate 14) | 3.00 % | 2.00 % | 3.00 % | |
| Diisostearoyl Polyglyceryl-3 Dimer Dilinoleate 15) | | 1.00 % | | |
| Cetyl PEG/PPG-10/1 Dimethicone 16) | | | | 2.00 % |
| Microcrystalline Wax | 0.10 % | 0.10 % | 0.10 % | 0.50 % |
| Hydrogenated Castor Oil | 0.10 % | 0.10 % | 0.10 % | 0.50 % |
| Diethylhexyl Carbonate | 7.00 % | 7.00 % | | |
| Triisostearin | 3.00 % | | | |
| Cylcoemethicone | 7.00 % | | | |
| C12-15 Alkyl Benzoate | | 7.00 % | | |
| Caprylic/Capric Triglyceride | | 6.80 % | | |
| Paraffinum Perliquidum (30 mPas) | | | 8.90 % | 10.50 % |
| Ethylhexyl Palmitate | | | 8.90 % | 10.50 % |
| **B (Water Phase)** | | | | |
| Skin Treatment Composition 1 (STC 1) | 5.00 % | | | 5.00 % |
| Skin Treatment Composition 2 (STC 4) | | 2.00 % | 2.00 % | |
| Water | 69.80 % | 69.50 % | 72.50 % | 67.50 % |
| Glycerin | 3.00 % | 3.00 % | 3.00 % | 3.00 % |
| Creatine | 1.00 % | | | |
| Magnesium Sulfate | 1.00 % | 1.50 % | 1.50 % | |
| Heptahydrate | | | | |
| Sodium Chloride | | | | 0.50 % |
| **Z** | | | | |
| Preservative, Parfum | q.s. | q.s. | q.s. | q.s. |
| | | | | |

(continued)

| Z | | | | |
|---|---|---|---|---|
| Consistency | Lotion | Lotion | Lotion | Lotion |
| 13) ISOLAN® GPS (Degusssa)<br>14) ISOLAN® PDI (Degusssa)<br>15) ABIL® EM 90 (Degusssa) | | | | |

**Claims**

**1.** A liquid and pumpable skin treatment composition containing:

A) 0. 1 to 10 % by weight of a mixture consisting of:

A1) 10 to 80 % by weight of a mixture of at least two ceramides with the proviso that at least one of these ceramides contains a long alkyl, alkenyl or acyl side chain with 20 to 26 carbon atoms and that less than 40 % by weight has a side chain larger than 26 carbon atoms;
A2) 10 to 45 % by weight of cholesterol;
A3) 10 to 45 % by weight of free fatty acids with C12 to C30 alkyl-, alkenyl, alkadienyl, alkatrienyl or alkapolyenyl chains or combinations thereof;

B) 0.5 to 10 % by weight of a nonionic emulsifier or a nonionic emulsifier mixture with a combined HLB value of 12 to 19;
C) ≥ 40 % by weight of water and optionally;
D) 0.1 to 10 % by weight consistency enhancers and optionally;
E) auxiliaries and additives

with the proviso that A) to E) must add up to 100 % by weight.

**2.** A liquid and pumpable skin treatment composition according to claim 1 with:

A1) 10 to 80 % by weight of a mixture of skin identical ceramides consisting of

A1a) a content > 30 % by weight of ceramides with a long alkyl, alkenyl or acyl chain with > C18, while the contents of very long, chain with > C26 acylceramides is ≤ 40% by weight;
A1b) a content of 1 to 30 % by weight of ceramides with a short alkyl chain with C4 to C8.

**3.** A liquid and pumpable skin treatment composition according to at least one of the claims 1 or 2 with:

A1) that consists of at least 3, but preferably 4 natural or skin-identical ceramides:

- 8 to 35 % by weight, preferably 9 to 15 % by weight of one or two Acyl Ceramides with a side chain > 27 Carbon atoms, wherein up to 45 % by weight of the total acyl ceramides is ceramide 9
- > 10 % by weight, preferably 25 to 40 % by weight of at least one phytosphingosine based Ceramide NP or AP, preferably Ceramide 3 (Cer NS), optionally
up to 30 % by weight of natural or skin-identical ceramides with a short alkyl side chain with C4 to C8, are added;

and containing D)

**4.** A liquid and pumpable skin treatment composition according to at least one of the claims 1 to 3 containing 0.2 to 8 % by weight of the ceramide / cholesterol / free fatty acid mixture A.

**5.** A liquid and pumpable skin treatment composition according to at least one of the claims 1 to 4 containing 1 to 8 % by weight of a nonionic emulsifier or a nonionic emulsifier mixture with a combined HLB value of 12 to 19

**6.** A liquid and pumpable skin treatment composition according to at least one of the claims 1 to 5 containing 0.1 to 6 % by weight consistency enhancers.

**7.** A liquid and pumpable skin treatment composition according to at least one of the claims 1 to 6 containing 0.5 to 5 % by weight of the ceramide / cholesterol / free fatty acid mixture A) and 4 to 6 % by weight of a nonionic emulsifier or a nonionic emulsifier mixture with a combined HLB value of 12 to 19 and 1 to 5 % by weight consistency enhancers and optionally auxiliaries and additives added up to 100 % by weight with water.

**8.** A liquid and pumpable skin treatment composition according to at least one of the claims 1 to 7 in which the ceramide mixture

A1) consists of the combination of ceramides:

- 50 to 70 % by weight of Ceramide NS + Ceramide NP in a 45/55 ratio with a side chain length of 20 to 26 C atoms; wherein upto 20 % by weight of the NS + NP may be replaced by Ceramide NH with a similar sidechain length;
- 14 to 20 % by weight of Ceramide EOS + Ceramide EOP in a 6 to 4 ratio; wherein upto 20 % by weight of the EOS + EOP may be replaced by Ceramide EOH with a similar side chain length;
- > 10 % by weight of Ceramide NP with a medium side chain length of 16 to 18 C atoms; wherein up to 30 % by weight of the Ceramide NP may be replaced by its Sphingosine [NS], 6-OH Sphingosine [NH} or Sphinganine [NSa] based equivalent;
- > 0 % by weight of Ceramide AP with a side chain length of 16 to 24 C atoms, wherein up to 30 % by weight of the Ceramide NP may be replaced by its Sphingosine [NS], 6-OH Sphingosine [NH} or Sphinganine [NSa] based equivalent;
- > 0 % by weight of Ceramide NS + NP with a side chain length of 4 to 8 C-atoms in a 1/1 ratio.

**9.** A liquid and pumpable skin treatment composition according to at least one of the claims 1 to 8 in which the free fatty acid components A3 are selected from a group of fatty acids with an alkyl or alkenyl chain of C14 to C28.

**10.** A liquid and pumpable skin treatment composition according to at least one of the claims 1 to 9 in which behenic acid is used as free fatty acid component A3).

**11.** A liquid and pumpable skin treatment composition according to at least one of the claims 1 to 10 in which the nonionic emulsifier or emulsifier mixture B) consists of a nonionic emulsifier or nonionic emulsifier mixture with a total HLB value of 14 to 18.

**12.** A liquid and pumpable skin treatment composition according to at least one of the claims 1 to 11 in which Ceteareth-25 is used as nonionic emulsifier component B).

**13.** A cosmetic, dermatological or pharmaceutical skin treatment formulation containing 0.001 to 20 by weight of a skin treatment composition according to at least one of the claims 1 to 12.

**14.** A cosmetic, dermatological or pharmaceutical skin treatment formulation according to claims 1 to 13 containing at least one component with additional skin benefit actives selected from the group consisting of vitamins, anti-oxidants, anti-inflammatories, anti-aging actives, anti-cellulite agents, moisturizers, sunscreens, skin lightening agents or skin tanning agents.

**15.** Hair care formulations containing a composition according to at least one of the claims 1 to 14.

**16.** "Wash-off" formulations containing a composition according to at least one of the claims 1 to 14.

**17.** Use of cosmetic formulations containing a composition according to at least one of the claims 1 to 14 for the treatment of dry skin.

**18.** Use of cosmetic formulations containing a composition according to at least one of the claims 1 to 14 for the treatment of aged skin.

**19.** Use of cosmetic formulations containing a composition according to at least one of the claims 1 to 14 for improving

the firmness of the skin.

## Patentansprüche

**1.** Flüssige und pumpfähige Hautbehandlungs-zusammensetzung, enthaltend:

A) 0,1 bis 10 Gewichtsprozent eines Gemischs, bestehend aus:

A1) 10 bis 80 Gewichtsprozent eines Gemischs von mindestens zwei Ceramiden, mit der Maßgabe, dass mindestens eines dieser Ceramide eine lange Alkyl-, Alkenyl- oder Acyl-Seitenkette mit 20 bis 26 Kohlenstoffatomen enthält, und dass mindestens 40 Gewichtsprozent eine Seitenkette haben, die größer als 26 Kohlenstoffatome ist;
A2) 10 bis 45 Gewichtsprozent Cholesterin;
A3) 10 bis 45 Gewichtsprozent freie Fettsäuren mit C12- bis C30-Alkyl-, -Alkenyl-, -Alkadienyl-, -Alkatrienyl- oder -Alkapolyenyl-Ketten oder Kombinationen davon;

B) 0,5 bis 10 Gewichtsprozent eines nichtionischen Emulgators oder eines nichtionischen Emulgatorgemischs mit einem kombinierten HLB-Wert von 12 bis 19;
C) ≥ 40 Gewichtsprozent Wasser und gegebenenfalls;
D) 0,1 bis 10 Gewichtsprozent Konsistenzverstärker und gegebenenfalls;
E) Hilfsstoffe und Additive
mit der Maßgabe, dass A) bis E) 100 Gewichtsprozent ausmachen müssen.

**2.** Flüssige und pumpfähige Hautbehandlungs-zusammensetzung nach Anspruch 1 mit:

A1) 10 bis 80 Gewichtsprozent eines Gemischs hautidentischer Ceramide, bestehend aus:

A1a) einem Gehalt von > 30 Gewichtsprozent Ceramiden mit einer langen Alkyl-, Alkenyl- oder Acylkette mit > C18, während der Inhalt der sehr langen Kette mit > C26 Acylceramiden ≤ 40 Gewichtsprozent ist;
A1b) einem Gehalt von 1 bis 30 Gewichtsprozent Ceramiden mit einer kurzen Alkylkette mit C4 bis C8.

**3.** Flüssige und pumpfähige Hautbehandlungs-zusammensetzung nach mindestens einem der Ansprüche 1 oder 2 mit:

A1), welches aus mindestens 3, aber vorzugsweise 4 natürlichen oder hautidentischen Ceramiden besteht;

- 8 bis 35 Gewichtsprozent, vorzugsweise 9 bis 15 Gewichtsprozent von einem oder zwei Acyl-Ceramiden mit einer Seitenkette > 27 Kohlenstoffatomen, wobei bis zu 45 Gewichtsprozent der Gesamt-Acyl-Ceramide Ceramid 9 sind;
- > 10 Gewichtsprozent, vorzugsweise 25 bis 40 Gewichtsprozent von mindestens einem Ceramid auf Phytosphingosin-Basis NP oder AP, vorzugsweise Ceramid 3 (Cer NS), und wobei gegebenenfalls

bis zu 30 Gewichtsprozent natürliche oder hautidentische Ceramide mit einer kurzen Alkylseitenkette mit C4 bis C8 zugegeben werden;

und welche D) enthält.

**4.** Flüssige und pumpfähige Hautbehandlungs-zusammensetzung nach mindestens einem der Ansprüche 1 bis 3, die 0,2 bis 8 Gewichtsprozent des Gemischs A aus Ceramid, Cholesterin und freier Fettsäure enthält.

**5.** Flüssige und pumpfähige Hautbehandlungs-zusammensetzung nach mindestens einem der Ansprüche 1 bis 4, die 1 bis 8 Gewichtsprozent eines nichtionischen Emulgators oder eines nichtionischen Emulgatorgemischs mit einem kombinierten HLB-Wert von 12 bis 19 enthält.

**6.** Flüssige und pumpfähige Hautbehandlungs-zusammensetzung nach mindestens einem der Ansprüche 1 bis 5, die 0,1 bis 6 Gewichtsprozent Konsistenzverstärker enthält.

**7.** Flüssige und pumpfähige Hautbehandlungs-zusammensetzung nach mindestens einem der Ansprüche 1 bis 6, die

0,5 bis 5 Gewichtsprozent des Gemischs A) aus Ceramid, Cholesterin und freier Fettsäure und 4 bis 6 Gewichtsprozent eines nichtionischen Emulgators oder eines nichtionischen Emulgatorgemischs mit einem kombinierten HLB-Wert von 12 bis 19 und 1 bis 5 Gewichtsprozent Konsistenzverstärker und gegebenenfalls Hilfsstoffe und Additive enthält, die mit Wasser auf 100 Gewichtsprozent aufgefüllt wird.

**8.** Flüssige und pumpfähige Hautbehandlungs-zusammensetzung nach mindestens einem der Ansprüche 1 bis 7, worin das Ceramid-Gemisch

A1) aus der Kombination der folgenden Ceramide besteht:

- 50 bis 70 Gewichtsprozent Ceramid NS + Ceramid NP in einem 45/55-Verhältnis mit einer Seitenkettenlänge von 20 bis 26 C-Atomen; wobei bis zu 20 Gewichtsprozent von NS + NP durch Ceramid NH mit einer ähnlichen Seitenkettenlänge ersetzt werden können;
- 14 bis 20 Gewichtsprozent Ceramid EOS + Ceramid EOP in einem 6 zu 4-Verhältnis; wobei bis zu 20 Gewichtsprozent von EOS + EOP durch Ceramid EOH mit einer ähnlichen Seitenkettenlänge ersetzt werden können;
- > 10 Gewichtsprozent Ceramid NP mit einer mittleren Seitenkettenlänge von 16 bis 18 C-Atomen; wobei bis zu 30 Gewichtsprozent des Ceramids NP durch sein Äquivalent auf Sphingosin [NS]-, 6-OH-Sphingosin [NH]- oder Sphinganin [NSa]-Basis ersetzt werden können;
- > 0 Gewichtsprozent Ceramid AP mit einer Seitenkettenlänge von 16 bis 24 C-Atomen, wobei bis zu 30 Gewichtsprozent des Ceramids NP durch sein Äquivalent auf Sphingosin [NS]-, 6-OH-Sphingosin [NH]- oder Sphinganin [NSa]-Basis ersetzt werden können;
- > 0 Gewichtsprozent Ceramid NS + NP mit einer Seitenkettenlänge von 4 bis 8 C-Atomen in einem 1/1-Verhältnis.

**9.** Flüssige und pumpfähige Hautbehandlungs-zusammensetzung nach mindestens einem der Ansprüche 1 bis 8, worin die freien Fettsäurekomponenten A3 aus einer Gruppe von Fettsäuren mit einer Alkyl- oder Alkenylkette von C14 bis C28 ausgewählt sind.

**10.** Flüssige und pumpfähige Hautbehandlungs-zusammensetzung nach mindestens einem der Ansprüche 1 bis 9, wobei Behensäure als freie Fettsäurekomponente A3) verwendet wird.

**11.** Flüssige und pumpfähige Hautbehandlungs-zusammensetzung nach mindestens einem der Ansprüche 1 bis 10, wobei der nichtionische Emulgator oder das nichtionische Emulgatorgemisch B) aus einem nichtionischen Emulgator oder nichtionischen Emulgatorgemisch mit einem Gesamt-HLB-Wert von 14 bis 18 besteht.

**12.** Flüssige und pumpfähige Hautbehandlungs-zusammensetzung nach mindestens einem der Ansprüche 1 bis 11, wobei Ceteareth-25 als nichtionische EmulgatorKomponente B) verwendet wird.

**13.** Kosmetische, dermatologische oder pharmazeutische Hautbehandlungsformulierung nach Anspruch 1 bis 13, die 0,001 bis 20 Gewichtsprozent einer Hautbehandlungs-Zusammensetzung nach mindestens einem der Ansprüche 1 bis 12 enthält.

**14.** Kosmetische, dermatologische oder pharmazeutische Hautbehandlungsformulierung nach Anspruch 1 bis 13, die mindestens eine Komponente mit zusätzlichen hautdienlichen Wirkstoffen enthält, ausgewählt aus der Gruppe, bestehend aus Vitaminen, Antioxidantien, Entzündungshemmern, Anti-Aging-Wirkstoffen, Anti-Cellulitis-Mitteln, Feuchtigkeitsmitteln, Sonnenschutzmitteln, Hautaufhellungsmitteln, oder Hautbräunungsmitteln.

**15.** Haarpflegeformulierungen, die eine Zusammensetzung nach mindestens einem der Ansprüche 1 bis 14 enthalten.

**16.** "Wash-off"-Formulierungen, d i e eine Zusammensetzung nach mindestens einem der Ansprüche 1 bis 14 enthalten.

**17.** Verwendung von kosmetischen Formulierungen, die eine Zusammensetzung nach mindestens einem der Ansprüche 1 bis 14 enthalten, zur Behandlung von trockener Haut.

**18.** Verwendung von kosmetischen Formulierungen, die eine Zusammensetzung nach mindestens einem der Ansprüche 1 bis 14 enthalten, zur Behandlung von gealterter Haut.

**19.** Verwendung von kosmetischen Formulierungen, die eine Zusammensetzung nach mindestens einem der Ansprüche 1 bis 14 enthalten, zur Verbesserung der Hautfestigkeit.

## Revendications

**1.** Composition de traitement de la peau liquide et pompable, contenant :

A) de 0,1 à 10% en poids d'un mélange constitué par :

A1) de 10 à 80% en poids d'un mélange d'au moins deux céramides, à condition qu'au moins l'un de ces céramides contienne une longue chaîne latérale alkyle, alcényle ou acyle ayant de 20 à 26 atomes de carbone et que moins de 40% en poids possède une chaîne latérale supérieure à 26 atomes de carbone ;
A2) de 10 à 45% en poids de cholestérol ;
A3) de 10 à 45% en poids d'acides gras libres ayant des chaînes alkyle, alcényle, alcadiényle, alcatriényle ou alcapolyényle en C12 à C30, ou des associations de celles-ci ;

B) de 0,5 à 10% en poids d'un émulsifiant non ionique ou d'un mélange d'émulsifiants non ioniques ayant une valeur combinée de rapport hydro-lipophile allant de 12 à 19 ;
C) 40% en poids d'eau ; et éventuellement
D) de 0,1 à 10% en poids d'agents améliorant la consistance ; et éventuellement
E) des auxiliaires et des additifs ;

à condition que A) à E) totalise jusqu'à 100% en poids.

**2.** Composition de traitement de la peau liquide et pompable selon la revendication 1, comprenant :

A1) de 10 à 80% en poids d'un mélange de céramides identiques à ceux de la peau, constitués

A1a) d'une teneur supérieure à 30% en poids de céramides ayant une longue chaîne alkyle, alcényle ou acyle supérieure à C18, tandis que la teneur en acylcéramides de chaîne très longue supérieure à C26 ests 40% en poids ;
A1b) d'une teneur allant de 1 à 30% en poids de céramides ayant une chaîne alkyle courte en C4 à C8.

**3.** Composition de traitement de la peau liquide et pompable selon au moins l'une des revendications 1 ou 2, avec :

A1) qui est constitué d'au moins 3, mais préférablement 4, céramides naturels ou identiques à ceux de la peau :

- de 8 à 35% en poids, préférablement de 9 à 15% en poids, d'un ou plusieurs acylcéramides ayant une chaîne latérale supérieure à 27 atomes de carbone, où jusqu'à 45% en poids des acylcéramides totaux sont constitués de céramide 9 ;
- > 10% en poids, préférablement de 25 à 40% en poids, d'au moins un céramide NP ou AP à base de phytosphingosine, préférablement le Céramide 3 (Cer NS), éventuellement

jusqu'à 30% en poids de céramides naturels ou identiques à ceux de la peau ayant une chaîne latérale alkyle courte de C4 à C8, sont ajoutés ; et contenant D).

**4.** Composition de traitement de la peau liquide et pompable selon au moins l'une des revendications 1 à 3, contenant de 0,2 à 8% en poids du mélange A de céramide/cholestérol/acides gras libres.

**5.** Composition de traitement de la peau liquide et pompable selon au moins l'une des revendications 1 à 4, contenant de 1 à 8% en poids d'un émulsifiant non ionique ou d'un mélange d'émulsifiants non ioniques ayant une valeur combinée de rapport hydro-lipophile allant de 12 à 19.

**6.** Composition de traitement de la peau liquide et pompable selon au moins l'une des revendications 1 à 5, contenant de 0,1 à 6% en poids d'agents améliorant la consistance.

**7.** Composition de traitement de la peau liquide et pompable selon au moins l'une des revendications 1 à 6, contenant

de 0,5 à 5% en poids du mélange A de céramide/cholestérol/acides gras libres, et de 4 à 6% en poids d'un émulsifiant non ionique ou d'un mélange d'émulsifiants non ioniques ayant une valeur combinée de rapport hydro-lipophile allant de 12 à 19, et de 1 à 5% en poids d'agents améliorant la consistance, et éventuellement des auxiliaires et des additifs ajoutés jusqu'à 100% en poids avec de l'eau.

**8.** Composition de traitement de la peau liquide et pompable selon au moins l'une des revendications 1 à 7, dans laquelle le mélange de céramides

A1) est constitué de la combinaison de céramides :

- de 50 à 70% de Céramide NS + Céramide NP selon un rapport 45/55 ayant une longueur de chaîne latérale allant de 20 à 26 atomes de C ; où jusqu'à 20% en poids du NS + NP peuvent être remplacés par du Céramide NH ayant une longueur de chaîne latérale similaire ;
- de 14 à 20% en poids de Céramide EOS + Céramide EOP selon un rapport allant de 6 à 4 ; où jusqu'à 20% en poids du EOS + EOP peuvent être remplacés par du Céramide EOH ayant une longueur de chaîne latérale similaire ;
- > 10% en poids de Céramide NP ayant une longueur de chaîne latérale moyenne allant de 16 à 18 atomes de C ; où jusqu'à 30% en poids du Céramide NP peuvent être remplacés par son équivalent à base de Sphingosine [NS], 6-OH-Sphingosine [NH] ou Sphingosine [NSa] ;
- > 0% en poids de Céramide AP ayant une longueur de chaîne latérale allant de 16 à 24 atomes de C ; où jusqu' à 30% en poids du Céramide NP peuvent être remplacés par son équivalent à base de Sphingosine [NS], 6-OH-Sphingosine [NH] ou Sphingosine [NSa] ;
- > 0% en poids de Céramide NS + NP ayant une longueur de chaîne latérale allant de 4 à 8 atomes de C selon un rapport de 1/1.

**9.** Composition de traitement de la peau liquide et pompable selon au moins l'une des revendications 1 à 8, dans laquelle les composants d'acides gras libres A3 sont choisis parmi le groupe constitué par les acides gras ayant une chaîne alkyle ou alcényle allant de C14 à C28.

**10.** Composition de traitement de la peau liquide et pompable selon au moins l'une des revendications 1 à 9, dans laquelle de l'acide béhénique est utilisé comme composant d'acides gras libres A3).

**11.** Composition de traitement de la peau liquide et pompable selon au moins l'une des revendications 1 à 10, dans laquelle l'émulsifiant ou le mélange d'émulsifiants B) non ioniques est constitué d'un émulsifiant non ionique ou d'un mélange d'émulsifiants non ioniques ayant une valeur totale de rapport hydro-lipophile allant de 14 à 18.

**12.** Composition de traitement de la peau liquide et pompable selon au moins l'une des revendications 1 à 11, dans laquelle du Ceteareth-25 est utilisé comme composant B) d'émulsifiant non ionique.

**13.** Formulation de traitement de la peau cosmétique, dermatologique ou pharmaceutique, contenant de 0,001 à 20% en poids d'une composition de traitement de la peau selon au moins l'une des revendications 1 à 12.

**14.** Formulation de traitement de la peau cosmétique, dermatologique ou pharmaceutique, selon les revendications 1 à 13, contenant au moins un composant ayant des substances actives supplémentaires ayant des effets bénéfiques sur la peau choisies parmi le groupe constitué par les vitamines, les antioxydants, les anti-inflammatoires, les substances actives antivieillissement, les agents anticellulite, les agents hydratants, les écrans solaires, les agents d'éclaircissement de la peau ou les agents de brunissement de la peau.

**15.** Formulations de soin des cheveux, contenant une composition selon au moins l'une des revendications 1 à 14.

**16.** Formulations à rincer, contenant une composition selon au moins l'une des revendications 1 à 14.

**17.** Utilisation de formulations cosmétiques contenant une composition selon au moins l'une des revendications 1 à 14, pour le traitement de la peau sèche.

**18.** Utilisation de formulations cosmétiques contenant une composition selon au moins l'une des revendications 1 à 14, pour le traitement de la peau âgée.

**19.** Utilisation de formulations cosmétiques contenant une composition selon au moins l'une des revendications 1 à 14, pour améliorer la fermeté de la peau.

Fig. 1

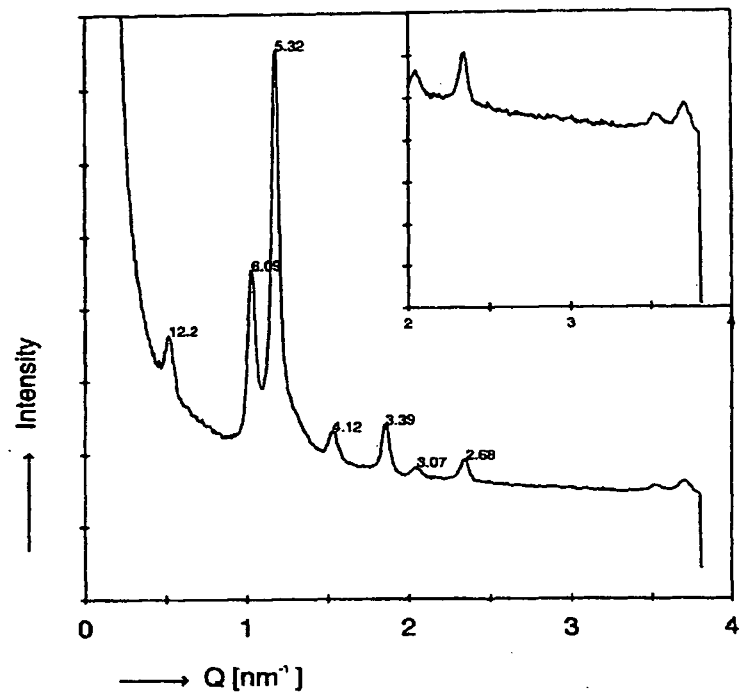

Fig. 2

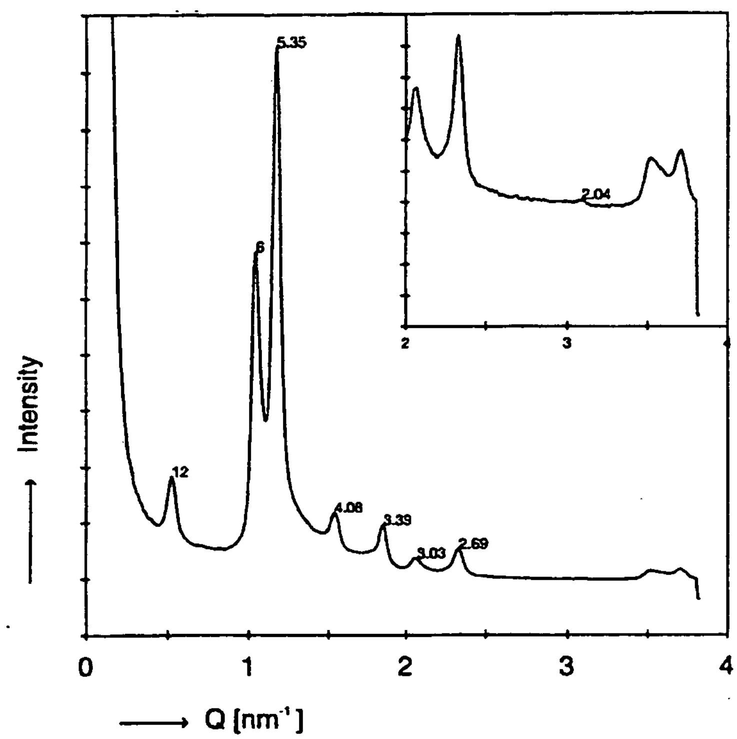

Fig. 3

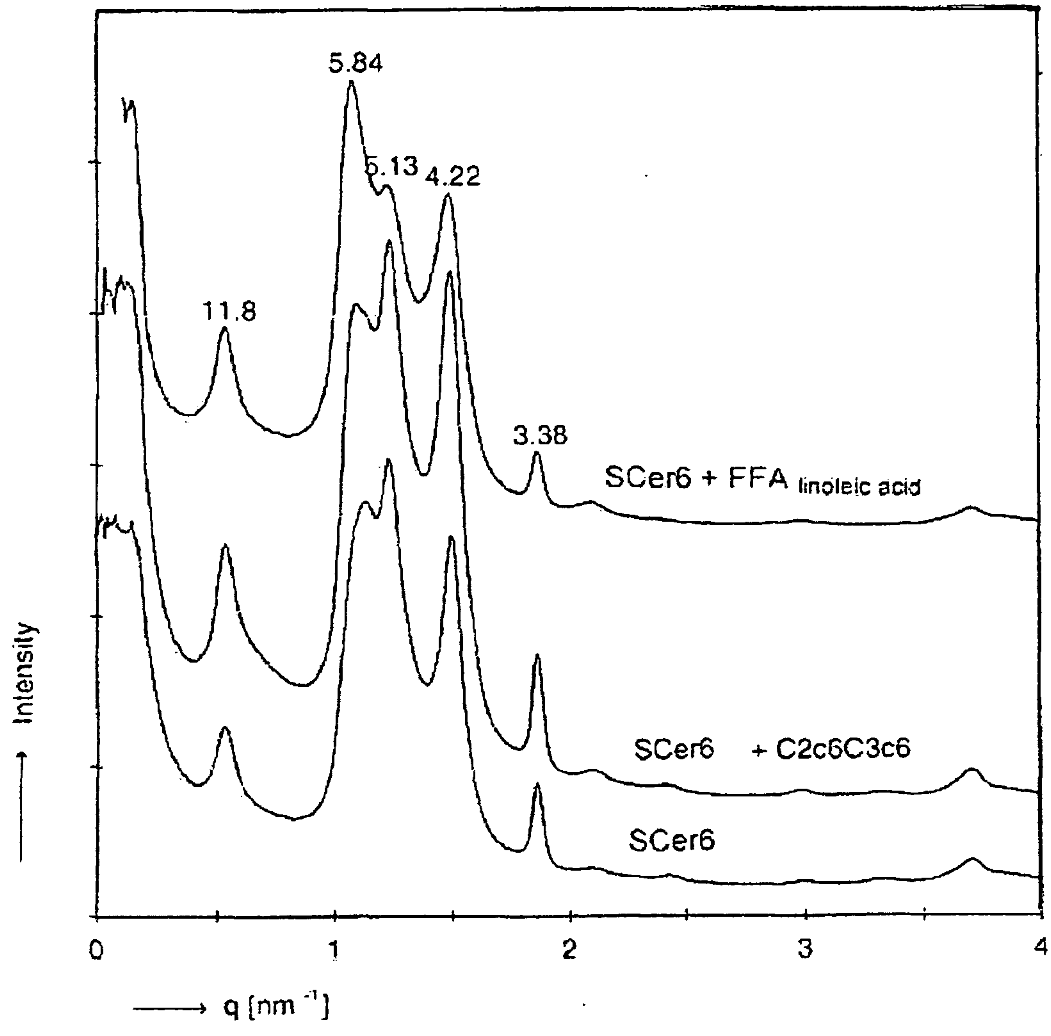

5.84
5.13
4.22
11.8
3.38
SCer6 + FFA linoleic acid
SCer6 + C2c6C3c6
SCer6
Intensity
0
1
2
3
4
q [nm ⁻¹]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0644764 A
- US 5578641 A, Jackson
- EP 0975325 A, Lambers
- DE 1165574
- DE 102005011785


### Non-patent literature cited in the description


- **H. Ziokowski GmbH ; Augsburg (D).** A Short Textbook of Cosmetology. Verlag, 1998
- **Elias PM.** *J. Invest. Dermatol.,* 1983, vol. 80 (1), 44
- **Rawlings AV.** *Int. J. Cosmet. Sci.,* 2003, vol. 25, 1-33
- **Rawlings, A.V. et al.** *J.Invest.Dermatol.,* 1994, vol. 103, 731-740
- **Motta S.** *Arch Dermatol.,* 1994, vol. 130, 452-456
- **Choi et al.** *J Invest Dermatol.,* March 2005, vol. 124 (3), 587-595
- **Zettersten.** *J Am Acad Dermatol.,* September 1997, vol. 37, 403-408
- **Beradesca.** *Contact Dermatitis,* 2001, vol. 45, 280-285
- **Pillai et al.** *J Invest Dermatol. Symp. Proceed.,* vol. 1, 39-93
- **Bouwstra J et al.** *Skin Pharmacol. Appl. Skin Physiol.,* 2001, vol. 14, 52-62
- **de Jager et al.** *J. Lipid Res.,* December 2005, vol. 46 (12), 2649-2656
- **Motta et al.** *BBA,* 1993, vol. 1182, 147-151
- **T. Dietz ; P. Hameyer.** *SOFW-Journal,* 2003, 2-9
- **Bouwstra, J.A. et al.** *J.Lipid Research,* 1998, vol. 39, 186-196
- **Bouwstra, J.A. et al.** *J. Invest. Dermatol.,* 2002, vol. 118, 606-617
- **de Jager,M.W. et al.** *Chem.Phys. Lipids,* 2003, vol. 124, 123-134
- **W.C. Griffin.** *J. Soc. Cosmet. Chem.,* 1949, vol. 1, 311
- **W.C. Griffin.** *J. Soc. Cosmet. Chem.,* 1954, vol. 5, 249
- Modern Aspects of Emulsion Science. The Royal Society of Chemistry, 1998
- **de Jager et al.** *J. Lipid Res.,* 2004, vol. 45, 923-932
- **de Jager et al.** *J. Lipid Res.,* 2005, vol. 46, 2649-2656
- **Bouwstra et al.** *J. Lipid Res.,* 1998, vol. 39, 186-196
- **Bouwstra et al.** *J. Invest. Dermatol.,* 2002, vol. 118, 606-617
- **De Jager et al.** *J. Lipid Res.,* 2005, vol. 46, 2649-2656